## Europäisches Patentamt

## European Patent Office

## Office européen des brevets

(19)

(11) Veröffentlichungsnummer : **0 079 516**
**B1**

(12)

# EUROPÄISCHE PATENTSCHRIFT

(45) Veröffentlichungstag der Patentschrift :
22.01.86

(51) Int. Cl.⁴ : **C 07 C119/048**, C 07 C118/00

(21) Anmeldenummer : **82110089.8**

(22) Anmeldetag : **02.11.82**

(54) Verfahren zur Herstellung von 4,4'-Diisocyanatodiphenylmethan.

(30) Priorität : **12.11.81 DE 3145010**

(43) Veröffentlichungstag der Anmeldung :
**25.05.83 Patentblatt 83/21**

(45) Bekanntmachung des Hinweises auf die Patenterteilung : **22.01.86 Patentblatt 86/04**

(84) Benannte Vertragsstaaten :
**BE DE FR GB IT NL**

(56) Entgegenhaltungen :
**US-A- 4 189 354**

(73) Patentinhaber : **BAYER AG**
**Konzernverwaltung RP Patentabteilung**
**D-5090 Leverkusen 1 Bayerwerk (DE)**

(72) Erfinder : **Ellendt, Günther, Dr.**
**Deswatinesstrasse 81**
**D-4150 Krefeld 1 (DE)**
Erfinder : **Gleitsmann, Günter, Dr.**
**Schönwasserstrasse 261**
**D-4150 Krefeld (DE)**
Erfinder : **Scheidel, Max, Dr.**
**St. Töniser Strasse 4**
**D-4156 Willich 1 (DE)**

EP 0 079 516 B1

0 079 516

**Beschreibung**

Die vorliegende Erfindung betrifft ein neues Verfahren zur destillativen Aufarbeitung von Polyisocyanatgemischen der Diphenylmethanreihe, wie sie durch Phosgenierung von Anilin/Formaldehyd-Kondensaten gewonnen werden, zwecks Herstellung von hochreinem 4,4'-Diisocanatodiphenylmethan.

Die durch Kondensation von Anilin und Formaldehyd in Gegenwart saurer Katalysatoren und nachfolgender Phosgenierung der so erhaltenen Polyamingemische resultierenden Polyisocyanatgemische der Diphenylmethanreihe enthalten neben den Isomeren und höheren Homologen des Diisocyanatodiphenylmethans stets wechselnde Mengen an Verunreinigungen wie z. B. an Monoisocyanaten, Farbkomponenten und Verbindungen mit organisch gebundenem Chlor. Ein Teil der letztgenannten Verbindungen enthält das Chlor in mehr oder weniger leicht abspaltbarer Form, sog. hydrolysierbares Chlor, daneben finden sich auch noch Verbindungen mit schwer abspaltbarem Chlor. Die unterschiedlichen Chlorverbindungen beeinflussen bekanntlich die Umsetzung von Isocyanaten mit Polyolen zu Polyurethanen, insbesondere wirken sie auf die Geschwindigkeit der Umsetzung ein. Vor allem bei dem zur Herstellung von elastischen Polyurethanen in großen Mengen eingesetzten 4,4'-Diisocyanatodiphenylmethan ist es erwünscht, die Wirkung der die Polyurethanreaktion beeinflussenden organischen Chlorverbindungen einzugrenzen, bzw. die Produkte bezüglich ihrer Chlorgehalte zu standardisieren. Der Gehalt an Chlorverbindungen ist somit eine wichtige Kenngröße für die Reinheit von 4,4'-Diisocyanatodiphenylmethan. Andererseits ist es erwünscht und erforderlich, den Gehalt an Monoisocyanaten wie z. B. das aus den Restgehalten von Anilin in den in die Phosgenierung eingesetzten Polyamingemischen entstammende Phenylisocyanat so niedrig wie möglich einzustellen, da Phenylisocyanat wegen seines niedrigen Dampfdrucks und hoher Toxizität bei der Verarbeitung und Handhabung der Isocyanate der Diphenylmethanreihe Probleme der Arbeitshygiene aufwirft.

Zur Erreichung des gewünschten Reinheitsgrades der Diisocyanatodiphenylmethane sind zwecks Entfernung der begleitenden Verunreinigungen eine Reihe von Verfahren bekannt geworden. So wurde z. B. vorgeschlagen, durch Zusatz von Stoffen, die chlorhaltigen Verbindungen in eine schwerflüchtige Form zu überführen (z. B. DE-PS 1 138 040) und die Begleitstoffe zu entfernen ; ein anderer Vorschlag (DE-OS 1 938 384) beruht auf der Anwendung eines technisch aufwendigen Kristallisationsverfahrens. Technisch breite Anwendung haben Verfahren zur destillativen Reinigung von Diisocyanatodiphenylmethanen gefunden. Bei den bisher bekannten Destillationsverfahren (vgl. z. B. DE-AS 2 631 168, DE-AS 1 923 214, US-PS 3 892 634 oder US-PS 3 471 543) erfolgt in der Regel als erster Destillationsschritt eine Abtrennung der Diisocyanatodiphenylmethan-Isomeren von ihren höheren Homologen. In einem zweiten Destillationsschritt wird die Trennung der isomeren Diisocyanatodiphenylmethane durchgeführt, wobei die leichter flüchtigen 2,2'- bzw. 2,4'-Isomeren als Kopfprodukte und das 4,4'-Isomere als Sumpfprodukt anfallen. Das von Isomeren weitgehend befreite 4,4'-Diisocyanatodiphenylmethan wird dann in einer Enddestillation zwecks Entfernung von Polymerisationsprodukten, die sich durch die thermische Belastung des beschriebenen Destillationsprozesses gebildet haben, nochmal abdestilliert.

Zur Abtrennung leicht bzw. schwer flüchtiger Verunreinigungen kann gemäß DE-AS 2 631 168 jeweils eine weitere Trennstufe zugeschaltet werden. Dieses Verfahren gestattet zwar, bei niedrigem Gehalt an Chlorverbindungen in dem in die Destillation eingespeisten Isocyanatgemisch auch niedrige Chlorgehalte im Endprodukt zu erreichen, jedoch gibt diese Verfahrensweise nicht genügend Sicherheit, auch bei erhöhten Chlorgehalten in dem eingespeisten Isocyanatgemisch die im Endprodukt erwünschten niedrigen Chlorgehalte zu erzielen.

Zur Einsparung von Destillationskolonnen wurde auch vorgeschlagen (DE-OS 2 933 601) den Schritt der Isomerentrennung und der Enddestillation durch Seitenstromentnahme in der gleichen Kolonne durchzuführen, wobei durch Einspeisen von Inertgas als Schleppmittel der Gehalt an Chlorverbindungen erniedrigt wird. Dieses Verfahren stellt aber keine Lösung des Problems dar, da hierbei Destillate mit Dimerengehalten in der Nähe der Sättigungsrenze ihrer Löslichkeit in Diisocyanatodiphenylmethan erhalten werden, d. h. es resultieren Produkte, die durch Ausfall von Dimeren entweder bereits bei der Destillation oder kurz danach trübe sind.

Durch das nachstehend näher beschriebene erfindungsgemäße Verfahren wird nun ein neuer Weg zu besonders reinem 4,4'-Diisocyanatodiphenylmethan gewiesen.

Gegenstand der Erfindung ist ein Verfahren zur Herstellung von 4,4'-Diisocyanatodiphenylmethan hoher Reinheit durch destillative Abtrennung von Diisocyanatodiphenylmethan-Isomeren aus einem durch Phosgenierung von Anilin/Formaldehyd-Kondensaten gewonnenen Polyisocyanatgemisch der Diphenylmethan-Reihe in einer Destillationsstufe (1), weitere Destillation des hierbei anfallenden Kopfprodukts in einer unter einem Rücklaufverhältnis von 0,1 : 1 bis 10 : 1 betriebenen Destillationsstufe (2), wobei 0,5-20 Gew.-% der in die Stufe (2) eingetragenen Produktmenge als Sumpf der Stufe (2) abgezogen wird, anschließende Abtrennung von 2,2'- und 2,4'-Diisocyanatodiphenylmethan aus der als Kopfprodukt der Stufe (2) erhaltenen Fraktion in einer nachgeschalteten, unter einem Rücklaufverhältnis von 2 : 1 bis 45 : 1 betriebenen Destillationsstufe (3) und destillative Aufarbeitung des in der Stufe (3) anfallenden Sumpfprodukts unter Gewinnung von hochreinem 4,4'-Diisocyanatodiphenylmethan, dadurch gekennzeichnet, daß man

a) die Temperaturen der Kondensatorausgänge der Destillationsstufe (1), (2) und (3) dergestalt auf

2

130 bis 230 °C einstellt, daß die Temperaturen 10 bis 50 °C unter der jeweils durch das Vakuum vorgegebenen Brüdentemperatur liegen, und

b) die Aufarbeitung des in der Stufe (3) anfallenden Sumpfes zweistufig dergestalt durchführt, daß man in einer ersten, unter einem

Rücklaufverhältnis von 0,2 : 1 bis 2 : 1 betriebenen Destillationsendstufe (4) 50 bis 90 Gew.-% des Sumpfes der Stufe (3) in Form von reinem 4,4'-Diisocyanatodiphenylmethan als Kopfprodukt isoliert und den Sumpf der ersten Endstufe (4) in einer zweiten, unter einem Rücklaufverhältnis von 0,2 : 1 bis 3 : 1 betriebenen Endstufe (4') in einen weiteren Anteil an reinem 4,4'-Diisocyanatodiphenylmethan als Kopfprodukt und einen Destillationsrückstand als Sumpf zerlegt.

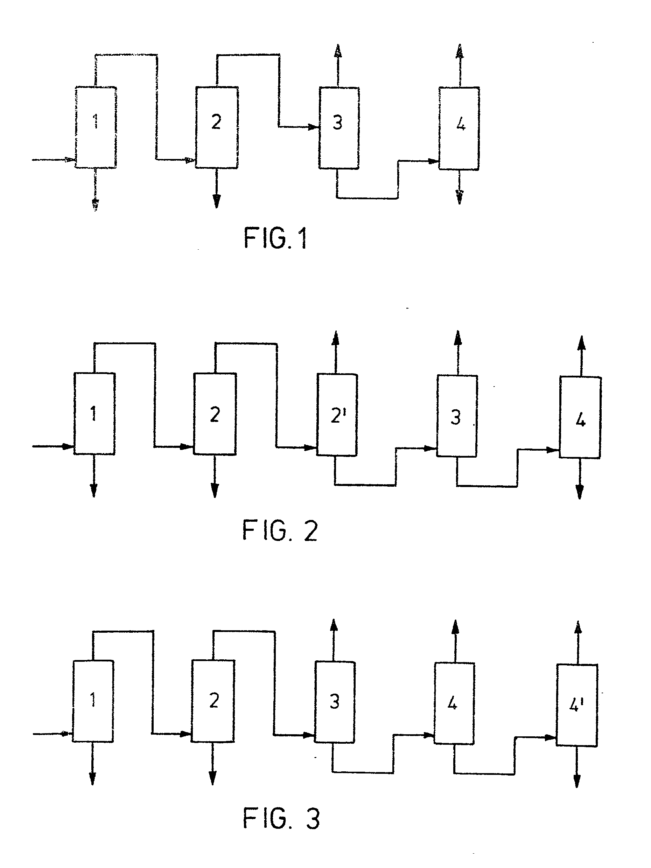

Das erfindungsgemäße Verfahren sei zunächst anhand der Zeichnungen näher erläutert. Hierbei entsprechen die Figuren 1 und 2 dem vorbekannten Verfahren gemäß DE-AS 2 631 168.

Figur 1 zeigt eine Ausführungsform zur destillativen Aufarbeitung von Polyisocyanatgemischen der Diphenylmethanreihe, gemäß welcher in einer Kolonne 1 aus einem Polyisocyanatgemisch der Diphenylmethanreihe neben als Sumpf anfallenden höheren Homologen ein Destillat erhalten wird, welches neben den 2,2'- und 2,4'-Isomeren das Hauptprodukt 4,4'-Diisocyanatodiphenylmethan enthält. Im Kopfprodukt sind noch Verunreinigungen enthalten, deren Siedepunkt dicht über bzw. unter denen der Diisocyanatodiphenyldimethan-Isomeren liegen. In der Kolonne (2) wird das Destillat erneut destilliert, wobei eine geringe Menge Sumpf ausgespeist wird, welcher bei der ersten Destillation mitgerissene Anteile, höher als Diisocyanatodiphenylmethan siedende Verunreinigungen, enthält. Das Destillat aus (2) wird in die Kolonne 3 eingespeist. Hier kann als Kopfprodukt ein überwiegend 2,4'-Diisocyanatodiphenylmethan enthaltendes Isomerengemisch erhalten werden. Das aus dem Sumpf von (3) ausgetragene vorgereinigte 4,4'-Diisocyanatodiphenylmethan wird anschließend in der Kolonne 4 durch Destillation von den durch die thermische Behandlung entstandenen Verunreinigungen befreit. Die Verunreinigungen werden mit einer kleineren Menge Sumpf ausgespeist. Das Destillat aus (4) stellt ein 4,4'-Diisocyanatodiphenylmethan mit eingestelltem Gehalt an Chlorverbindungen dar.

Bei diesem Verfahren ist der Gehalt an leichtsiedenden, insbesondere chlorhaltigen Verbindungen im Destillat der Kolonne (3) noch hoch. Dies überrascht nicht, da es sich bei DE-AS 2 631 168 um ein Verfahren zur Einstellung der Chlorgehalte handelt. Aufgrund des hier erzielten Reinheitsgrades des Destillats aus (5) muß angenommen werden, daß die Kondensatorablauftemperaturen der Kolonnen 1-3 < 120 °C liegen.

Figur 2 zeigt eine verbesserte Ausführungsform des Verfahrens gemäß Stand der Technik. Hier wird durch Zuschalten einer Kolonne 2' zwischen die Kolonnen 2 und 3 an der Kolonne 2' ein Kopfprodukt entnommen, welches neben 2,2'- und 2,4'-Diisocyanatodiphenylmethan vorwiegend Verbindungen mit Siedepunkten niedriger als die Diisocyanatodiphenylmethanisomere enthält, so daß im Destillat der Kolonne 3 niedrige Chlorgehalte feststellbar sind. Diese Variation des Verfahrens des Standes der Technik liefert allerdings im Destillat der Kolonne 4 immer noch mehr oder weniger hohe Chlorgehalte.

Figur 3 dient zur näheren Erläuterung des neuen erfindungsgemäßen Verfahrens.

In die Destillationsstufe (1) wird ein Polyisocyanatgemisch der Diphenylmethanreihe eingespeist und in ein höhere Homologen enthaltendes Sumpfprodukt und ein rohes Diisocyanatodiphenylmethan-Isomerengemisch aufgetrennt. Die Destillation in der Destillationsstufe (1) stellt im allgemeinen eine einfache Destillation ohne Rücklauf dar. Selbstverständlich könnte die Destillation in der Stufe (1) auch unter Verwendung einer Destillationskolonne unter Rückfluß stattfinden, obwohl dies, wie gesagt, nicht erforderlich ist. Die Kondensation des Destillats erfolgt, wie oben ausgeführt, so, daß die Temperatur des Kondensatorausgangs bei 130-230 °C, vorzugsweise 130-200 °C und insbesondere bei 140-160 °C liegt. Die Menge in der Stufe (1) anfallenden Destillats ist nicht kritisch ; sie hängt von der Zusammensetzung des Polyisocyanatgemischs und insbesondere davon ab, welche Viskosität der in der Stufe (1) anfallende Rückstand aufweisen soll.

In der der Destillationsstufe (1) nachgeschalteten, aus einer oder mehreren Destillationskolonnen bestehenden Destillationsstufe (2) erfolgt eine destillative Abtrennung von höher als die Diisocyanatodiphenylmethan-Isomeren siedenden Verunreinigungen des Destillats der Stufe (1). Die Destillationsstufe (2) wird unter einem Rücklaufverhältnis (Rücklaufverhältnis = Gewichtsverhältnis von Rücklauf zur Destillatentnahme) von 0,1 : 1 bis 10 : 1, vorzugsweise 0,3 : 1 bis 3 : 1 betrieben. Aus dem Sumpf der Stufe (2) werden kontinuierlich 0,5 bis 20, vorzugsweise 1 bis 10 Gew.-% der in die Stufe (2) eingetragenen Menge ausgeschleust. Die Hauptmenge wird über den Kopf der Stufe (2) destilliert, wobei die Kondensationstemperatur den bereits oben gemachten Ausführungen entspricht.

In der der Stufe (2) nachgeschalteten, ebenfalls aus einer oder mehreren Destillationskolonnen bestehenden Destillationsstufe (3) erfolgt eine destillative Abtrennung der im Destillat der Stufe (2) enthaltenen 2,2'- und 2,4'-Isomeren. Die Destillationsstufe (3) wird unter einem Rücklaufverhältnis von 2 : 1 bis 45 : 1, vorzugsweise 15 : 1 bis 30 : 1 betrieben. Die Kondensationstemperatur wird auch hier, wie bereits oben erläutert, eingestellt.

Das als Sumpf der Destillationsstufe (3) anfallende vorgereinigte 4,4'-Diisocyanatodiphenylmethan wird schließlich in den Endstufen 4 und 4' destillativ gereinigt. Die Destillation in der Destillationsstufe (3) wird so geführt, daß der anfallende Sumpf maximal 3 Gew.-% 2,4'-Diisocyanatodiphenylmethan enthält.

Zur destillativen Reinigung des Sumpfes der Stufe (3) werden in der 1. Endstufe (4) 50 bis 90 Gew.-% des in der Stufe (3) anfallenden Sumpfes in Form von reinem 4,4'-Diisocyanatodiphenylmethan über Kopf

3

abdestilliert. Diese erste Endstufe (4) wird unter einem Rücklauf von 0,2 : 1 bis 2 : 1 betrieben. Das weniger als 5 ppm Phenylisocyanat und weniger als 5 ppm an hydrolisierbarem Chlor enthaltende Destillat wird vorzugsweise unmittelbar nach Verlassen der ersten Endstufe (4) auf unter 50 °C abgekühlt. Auch die erste Endstufe (4) kann im Prinzip aus einer oder mehreren, in Serie geschalteten Destillationskolonnen bestehen, wobei jeweils der Sumpf der vorgeschalteten Kolonne in die nachgeschaltete Kolonne eingespeist wird. Die o. g. Bedingung, daß in der ersten Stufe (4) 50 bis 90 Gew.-% des in der Stufe (3) anfallenden Sumpfes in Form von reinem 4,4'-Diisocyanatodiphenylmethan über Kopf abdestilliert wird, bezieht sich hierbei auf die Summe aller Kopfprodukte der der letzten Stufe (4') vorgeschalteten Kolonnen der (gegebenenfalls mehrere, in Serie geschaltete Kolonne aufweisenden) Stufe (4).

Der Sumpf der ersten Endstufe (4), d. h. im Falle der Verwendung einer einzigen Destillationskolonne der Sumpf dieser Kolonne, bzw. im Falle der Verwendung mehrerer in Serie geschalteter Kolonnen der Sumpf der letzten dieser Kolonnen wird in die letzte Endstufe (4') eingetragen und dort in eine zweite Menge an reinem 4,4'-Diisocyanatodiphenylmethan als Kopfprodukt und ca. 5 bis 15 Gew.-%, bezogen auf die Gesamtmenge des Sumpfes aus (4) an Sumpfprodukt zerlegt. Das Rücklaufverhältnis der letzten, aus einer einzigen Destillationskolonne bestehenden Endstufe (4') liegt bei 0,2 : 1 bis 3 : 1, vorzugsweise 0,2 : 1 bis 1 : 1. Die Qualität des als Kopfprodukt erhaltenen 4,4'-Diisocyanatodiphenylmethans entspricht der Qualität des Kopfprodukts der Stufe (4). Auch hier wird das Kopfprodukt vorzugsweise unmittelbar nach Verlassen der Kolonne auf unter 50 °C abgekühlt. Durch diese Abkühlung wird die Bildung von Dimeren weitgehend ausgeschlossen. Der in der letzten Endstufe (4') anfallende Sumpf enthält die durch die thermische Belastung gebildeten Verunreinigungen, sowie solche chlorhaltigen Verunreinigungen, die einen höheren Siedepunkt aufweisen als das 4,4'-Diisocyanatodiphenylmethan.

Gemäß einer besonderen Ausführungsform des erfindungsgemäßen Verfahrens kann die in den Kondensatoren der Destillationsstufen anfallende Kondensationswärme zur Rückgewinnung von Dampf einer Druckstufe von 2 bis 16 bar ausgenutzt werden, wozu die Kondensatoren mit Wasser unter einem solchen Druck betrieben werden, der sich aus der gewählten Ablauftemperatur der Kondensate ergibt.

Die beim erfindungsgemäßen Verfahren zum Einsatz gelangenden Destillationsstufen können mit Ausnahme der letzten Stufe (4') jeweils aus einer oder mehreren Destillationseinheiten bestehen, wobei insbesondere im Falle der Destillationsstufen (2), (3), (4) und (4') trennwirksame Destillationskolonnen zum Einsatz gelangen. Bei den Destillationsstufen (1) bis (3) liegt die Temperatur der Kondensatorausgänge innerhalb der genannten Bereiche, d. h. zwischen 130 und 230 °C, vorzugsweise 130 bis 200 °C und insbesondere 140 bis 160 °C, wobei stets die zweite Bedingung, daß die Temperatur der Kondensatorausgänge 10 bis 50 °C, vorzugsweise 20 bis 40 °C unter der jeweils durch das Vakuum vorgegebenen Brüdentemperatur liegt, gleichzeitig erfüllt sein muß. Auch die Destillationsstufen (4) und (4') können unter Einhaltung dieser Bedingungen betrieben werden, wobei jedoch die vorzugsweise durchzuführende Abkühlung der Destillate auf unterhalb 50 °C unmittelbar am Ausgang der Kondensatoren erfolgt.

Das erfindungemäße Verfahren weist gegenüßer dem nächstliegenden Verfahren des Standes der Technik gemäß DE-AS 2 631 168 insbesondere folgender Vorteile auf :

a) Durch die Einstellung der Produktablauftemperaturen auf Werte oberhalb 130 °C wird erreicht, daß leichtflüchtige Verunreinigungen wie Phenylisocyanat oder Verbindungen mit organisch gebundenem Chlor aus den Destillaten entgasen können und so über das Vakuumsystem entfernt werden. Hierdurch wird die Herstellung von hochreinem 4,4'-Diisocyanatodiphenylmethan unter Einsparung der Destillationsstufe 2' (Figur 2) ermöglicht.

b) Da es sich bekanntlich bei der destillativen Reinigung von Diisocyanatodiphenylmethanen nicht um einfache destillative Auftrennung bzw. Abtrennung von Verunreinigungen handelt, sondern vielmehr, bedingt durch die thermische Belastung der Produkte, Zersetzungen des Isocyanates stattfinden, die zu Ausbeuteverlusten führen, bzw. organisch gebundenes Chlor enthaltende Verunreinigungen, gespalten und zu anderen Verbindungen, z. T. mit höheren Siedepunkten, rekombiniert werden können, treten nach den Verfahren des Standes der Technik im Destillat der Kolonne 4 (Fig. 2) schwankende Gehalte an hydrolysierbarem Chlor auf, wobei die Gehalte mit steigendem Chlorniveau im Einlauf der vorgeschalteten Kolonnen höher werden. Durch das erfindungsgemäße Verfahren wird ein 4,4'-Diisocyanatodiphenylmethan erhalten, welches konstante Chlorgehalte auf extrem niedrigem Niveau aufweist.

Um Chlorverbindungen mit Siedepunkten dicht über dem des 4,4'-Diisocyanatodiphenylmethans abzutrennen und die Ausbeute an 4,4'-Diisocyanatodiphenylmethan zu verbessern wird entsprechend dem erfindungsgemäßen Verfahren die Destillation des vorgereinigten 4,4'-Diisocyanatodiphenylmethans in einer zweistufigen Destillation durchgeführt. Hierbei handelt es sich nicht um eine zweite Destillationsstufe, vielmehr werden in der ersten Stufe 4 bei stark verminderter Verweilzeit im Sumpf und damit verminderter thermischer Belastung 50 bis 90 % der eingespeisten Menge abdestilliert und 10 bis 50 % Sumpf ausgespeist. Die große Menge ausgespeisten Sumpfes bewirkt zusammen mit dem Rücklaufverhältnis von 0,2 : 1 bis 2 : 1 eine hervorragende Abtrennung der Chlorverbindungen bei gleichzeitiger Vermeidung der Neubildung von Verunreinigungen. In der nachgeschalteten Stufe 4' erfolgt dann das Abdestillieren des restlichen 4,4'-Diisocyanatodiphenylmethans, wobei 5-15 % des Einlaufmenge als Sumpf ausgetragen werden. In der Kolonne 4' ist die Verweilzeit und somit die thermische Belastung des 4,4'-Diisocyanatodiphenylmethans ebenfalls geringer als beim Verfahren des

**0 079 516**

Standes der Technik, so daß hier im Destillat nicht mehr als 5 ppm an hydrolysierbarem Chlor erhalten werden können.

Die bei dem erfindungsgemäßen Verfahren erhaltenen Destillate der Stufen 3, 4 und 4' sind von einem extremen Reinheitsgrad, insbesondere im Hinblick auf den Gehalt an Phenylisocyanat, Verbindungen mit organisch gebundenem Chlor sowie von Begleitstoffen, die zur Verfärbung unter Licht- und Lufteinwirkung führen. Ein besonderer Vorteil des Verfahrens ist es, daß dieser Reinheitsgrad auch bei schwankenden Gehalten an Verunreinigungen in dem als Ausgangsprodukt eingesetzten Isocyanatgemisch erreicht werden kann. Darüber hinaus kann bei den erzeugten Produkten vielfach auf den Zusatz von Stabilisatoren, die gegen Verfärbungen schützen, verzichtet werden.

Die folgenden Beispiele sollen das erfindungsgemäße Verfahren näher erläutern.

Alle Prozentangaben beziehen sich auf Gewichtsprozente. Die nachfolgenden Beispiele 1 und 2 stellen Vergleichsbeispiele dar. Die Temperaturen der Kondensatorausgänge der Stufen (1), (2), (2') und (3) liegen bei jeweils ca. 100-110 °C bei einer Brüdentemperatur Temperatur von ca. 180 °C.

Beispiel 1 (Tab. 1)

Bisheriges Verfahren mit Abtrennung von Produkten mit Siedepunkten die höher liegen als der des Diisocyanatodiphenylmethans.

Ein Polyisocyanatgemisch der Diphenylmethanreihe, welches etwa

5,6 % 2,4'-Diisocyanatodiphenylmethan
80,4 % 4,4'-Diisocyanatodiphenylmethan
12,8 % 3-Kern-Verbindungen
1,2 % 4- und höher-kernige Verbindungen

enthält, wird in einer kontinuierlich arbeitenden Destillationsanlage mit den Teilen 1, 2, 3 und 4 (Tab. 1) aufgearbeitet. 1, 2, 3 und 4 sind Destillationseinheiten bestehend aus Umlaufverdampfern mit entsprechenden Kreislaufpumpen, Kondensatoren für das Destillat und Anschluß an ein geeignetes Vakuumsystem. Bei 2, 3 und 4 ist zwischen Verdampfer und Kondensator eine Kolonne geschaltet.

Die bei unterschiedlicher Einlaufmenge und Qualität des in die Destillationseinheit 1 eingespeisten Produktes in den Destillaten erzielbaren Chlorgehalte stellen sich wie folgt dar.

(Siehe Tabelle 1 Seite 6 f.)

5

## Beispiel 1 (Tab. 1)

Verfahren mit Abtrennung von höher als Diisocyanatodiphenylmethan-Isomere (MDI) siedenden Verunreinigungen

| Destillat aus Kolonne | Menge kg/h | Rücklauf kg/h | hydrolysierbares Chlor ppm | Gesamtchlor ppm |
|---|---|---|---|---|
| 1 | 1.255 | – | 359 | 741 |
|   | 950 | – | 240 | 570 |
| 2 | 1.715 | 800 | 152 | 360 |
|   | 850 | 800 | 160 | 280 |
| 3 | 178 | 2.000 | 214 | 450 |
|   | 75 | 1.200 | 115 | 280 |
| 4 | 1.010 | 800 | 25 | 60 |
|   | 740 | 800 | 4 | 12 |

## Beispiel 2 (Tab. 2)

Wie Beispiel 1 jedoch ergänzt um eine Destillationsstufe 2' zur Abtrennung leicht flüchtiger Begleitstoffe.

Zur Abtrennung von Nebenprodukten mit Siedepunkten unter dem des 4,4'-Diisocyanatodiphenylmethans wurde eine weitere Stufe 2' zugeschaltet, welche wie 3 aus Verdampfer, Kolonnenpackung und Kondensator besteht.

Die in der Tabelle aufgelisteten Daten geben die Qualität der Destillationsabläufe in Form der gemessenen Chlorgehalte wieder.

(Siehe Tabelle 2 Seite 8 f.)

Beispiel 2 (Tab. 2)

Verfahren mit Abtrennung von höher und niedriger als MDI siedenden Verunreinigungen

| Destillat aus Kolonne | Menge kg/h | Rücklauf kg/h | hydrolysierbares Chlor ppm | Gesamtchlor ppm |
|---|---|---|---|---|
| 1 | 1.690 | – | 460 | 820 |
|   | 1.200 | – | 280 | 530 |
| 2 | 1.650 | 1.200 | 250 | 410 |
|   | 1.170 | 1.200 | 170 | 320 |
| 2' | 50 | 2.000 | 2.400 | 3.760 |
|   | 45 | 2.000 | 1.640 | 2.985 |
| 3 | 230 | 4.000 | 4 | 20 |
|   | 170 | 4.000 | 10 | 35 |
| 4 | 1.330 | 1.200 | 29 | 55 |
|   | 925 | 1.200 | 5 | 18 |

0 079 516

# 0 079 516

Beispiel 3 (Tab. 3)

Erfindungsgemäßes Verfahren

Ein Polyisocyanatgemisch der Zusammensetzung gemäß Beispiel 1 wird einer kontinuierlich betriebenen Destillationsanlage, bestehend aus den Einheiten 1, 2, 3, 4, 4' (Abb. 2) aufgearbeitet.

Die Teile 1, 2, 3, 4 und 4' sind Destillationsstufen bestehend aus Umlaufverdampfern mit den entsprechenden Kreislaufpumpen, Kondensatoren für das Destillat in welchen durch Kühlung mit Wasser unter entsprechendem Druck die Kondensationswärme zur Erzeugung von Dampf mit einer Druckstufe von 2-6 bar genutzt werden kann, sowie Anschluß an ein geeignetes Vakuumsystem. Bei 2, 3, 4 und 4' ist zwischen Verdampfer und Kondensator eine Kolonne geschaltet. Die Kondensatoren der Einheiten 1 und 4' ermöglichen ein rasches Abschrecken des Destillates auf Temperaturen von 45-50 °C.

Mit dieser Anordnung wurden die folgenden Ablaufqualitäten erhalten.

(Siehe Tabelle 3 Seite 10 f.)

9

Beispiel 3 (Tab. 3)

Destillation mit Kondensatablauftemperaturen in den Kolonnen 1, 2 und 3 von 145 °C (bei einer Brüdentemperatur von 180 °C) und zweistufiger Enddestillation

| Destillatablauf aus Kolonne | Menge kg/h | Rücklauf kg/h | hydrolysierbares Chlor ppm | Gesamtchlor ppm |
|---|---|---|---|---|
| 1 | 1.850 | | 390 | 760 |
| | 1.040 | | 220 | 480 |
| 2 | 1.545 | 1.150 | 230 | 390 |
| | 1.000 | 1.200 | 150 | 240 |
| 3 | 215 | 4.000 | 6 | 21 |
| | 135 | 3.900 | 8 | 37 |
| 4 | 995 | 1.200 | 4 | 10 |
| | 600 | 1.350 | 5 | ·15 |
| 4' | 300 | 800 | 5 | 20 |
| | 230 | 970 | 5 | 17 |

0 079 516

**Patentansprüche**

1. Verfahren zur Herstellung von 4,4'-Diisocyanatodiphenylmethan hoher Reinheit durch destillative Abtrennung von Diisocyanatodiphenylmethan-Isomeren aus einem durch Phosgenierung von Anilin/Formaldehyd-Kondensaten gewonnenen Polyisocyanatgemisch der Diphenylmethan-Reihe in einer Destillationsstufe (1), weitere Destillation des hierbei anfallenden Kopfprodukts in einer unter einem Rücklaufverhältnis von 0,1 : 1 bis 10 : 1 betriebenen Destillationsstufe (2), wobei 0,5-20 Gew.-% der in die Stufe (2) eingetragenen Produktmenge als Sumpf der Stufe (2) abgezogen wird, anschließende Abtrennung von 2,2' und 2,4'-Diisocyanatodiphenylmethan aus der als Kopfprodukt der Stufe (2) erhaltenen Fraktion in einer nachgeschalteten, unter einem Rücklaufverhältnis von 2 : 1 bis 45 : 1 betriebenen Destillationsstufe (3) und destillative Aufarbeitung des in der Stufe (3) anfallenden Sumpfprodukts unter Gewinnung von hochreinem 4,4'-Diisocyanatodiphenylmethan, dadurch gekennzeichnet, daß man

a) die Temperaturen der Kondensatorausgänge der Destillationsstufe (1), (2) und (3) dergestalt auf 130 bis 230 °C einstellt, daß die Temperaturen 10 bis 50 °C unter der jeweils durch das Vakuum vorgegebenen Brüdentemperatur liegen, und

b) die Aufarbeitung des in der Stufe (3) anfallenden Sumpfes zweistufig dergestalt durchführt, daß man in einer ersten, unter einem Rücklaufverhältnis von 0,2 : 1 bis 2 : 1 betriebenen Destillationsendstufe (4) 50 bis 90 Gew.-% des Sumpfes der Stufe (3) in Form von reinem 4,4'-Diisocyanatodiphenylmethan als Kopfprodukt isoliert und den Sumpf der ersten Endstufe (4) in einer zweiten, unter einem Rücklaufverhältnis von 0,2 : 1 bis 3 : 1 betriebenen Endstufe (4') in einen weiteren Anteil an reinem 4,4'-Diisocyanatodiphenylmethan als Kopfprodukt und einen Destillationsrückstand als Sumpf zerlegt.

2. Verfahren gemäß Anspruch 1, dadurch gekennzeichnet, daß man die Destillate der Endstufen (4) und (4') rasch auf unterhalb 50 °C abkühlt.

3. Verfahren gemäß Anspruch 1 und 2, dadurch gekennzeichnet, daß man die Kondensationswärme der anfallenden Destillate und Rückläufe zur Rückgewinnung von Dampf der Druckstufe von 2 bis 16 ausnutzt.

**Claims**

1. Process for the production of 4,4'-diisocyanatodiphenylmethane of high purity by the distillative separation of diisocyanatodiphenylmethane isomers from a polyisocyanate mixture of the diphenylmethane series obtained by phosgenating aniline/formaldehyde condensates, in a distillation stage (1), further distillation of the top product thereby obtained in a distillation stage (2) operated with a reflux ratio of 0.1 : 1 to 10 : 1, 0.5-20 % by weight of the quantity of product introduced into stage (2) being withdrawn as the bottom product of stage (2), subsequent separation of 2,2'- and 2,4'-diisocyanatodiphenylmethane from the fraction obtained as the top product of stage (2) in a downstream distillation stage (3) operated with a reflux ratio of 2 : 1 to 45 : 1 and distillative working up of the bottom product obtained in stage (3) with the obtainment of highly pure 4,4'-diisocyanatodiphenylmethane, characterised in that

a) the temperatures of the condenser outlets of the distillation stage (1), (2) and (3) are adjusted to 130 to 230 °C such that the temperatures are 10 to 50 °C below the vapour temperature predetermined in each case by the vacuum, and

b) the working up of the bottom product obtained in stage (3) is carried out in two stages, in such a manner that, in a first distillation end stage (4) operated with a reflux ratio of 0.2 : 1 to 2 : 1, 50 to 90 % by weight of the bottom product stage (3) are isolated as the top product in the form of pure 4,4'-diisocyanatodiphenylmethane and the bottom product of the first end stage (4) is split, in a second end stage (4') operated with a reflux ratio of 0.2 : 1 to 3 : 1, into a further quantity of pure 4,4'-diisocyanatodiphenylmethane as the top product and a distillation residue as the bottom product.

2. Process according to Claim 1, characterised in that the distillates of the end stages (4) and (4') are cooled rapidly to below 50 °C.

3. Process according to Claim 1 and 2, characterised in that the heat of condensation of the distillates and refluxes obtained is utilised for the recovery of vapour of a pressure stage of 2 to 16 bar.

**Revendications**

1. Procédé de préparation d'un 4,4'-diisocyanatodiphénylméthane à haute pureté par séparation par distillation des isomères du diisocyanatodiphénylméthane à partir d'un mélange de polyisocyanates de la série du diphénylméthane obtenu par phosgénation de condensats aniline/formaldéhyde dans un stade de distillation (1), nouvelle distillation du produit de tête ainsi obtenu dans un stade de distillation (2) opérant avec un raport de reflux de 0,1 : 1 à 10 : 1, avec évacuation en culot du stade 2 de 0,5 à 20 % en poids de la quantité de produit introduite au stade (2), séparation subséquente du 2,2'- et du 2,4'-diisocyanatodiphénylméthane à partir de la fraction obtenue en produit de tête du stade (2) dans un stade de distillation (3) placé à la suite et opérant avec un rapport de reflux de 2 : 1 à 45 : 1, et traitement par distillation du produit de culot obtenu au stade (3) avec obtention d'un 4,4'-diisocyanatodiphénylméthane à haute pureté, caractérisé en ce que

a) on règle les températures des sorties de condenseurs des stades de distillation (1), (2) et (3) à un niveau de 130 à 230 °C, ces températures se situant à un niveau de 10 à 50 °C au-dessous de la température des vapeurs déterminée dans chaque cas par le vide, et

b) on procède·au traitement du culot obtenu au stade (3) en deux stades : dans un premier stade final de distillation (4) opérant avec un rapport de reflux de 0,2 : 1 à 2 : 1, on isole en produit de tête de 50 à 90 % en poids du culot du stade (3) sous forme de 4,4'-diisocyanatodiphénylméthane pur et on fractionne le culot du premier stade final (4) dans un deuxième stade final (4') opérant avec un rapport de reflux de 0,2 : 1 à 3 : 1 en une autre fraction de 4,4'-diisocyanatodiphénylméthane pur en produit de tête et un résidu de distillation en culot.

2. Procédé selon la revendication 1, caractérisé en ce que l'on refroidit rapidement à une température inférieure à 50 °C les distillats des stades finals 4 et 4'.

3. Procédé selon les revendications 1 et 2, caractérisé en ce que l'on exploite la chaleur de condensation des distillats et des reflux pour la récupération de vapeur à un niveau de pression de 2 à 16 bars.

FIG.1

FIG. 2

FIG. 3

1